# EUROPÄISCHE PATENTSCHRIFT

(11) **EP 3 060 538 B1**
(45) Veröffentlichungstag und Bekanntmachung des Hinweises auf die Patenterteilung: **01.01.2020**
(21) Anmeldenummer: 14783853.6
(22) Anmeldetag: 13.10.2014
(51) Int. Cl.: C07C 29/88, C07C 31/20

(54) **VERFAHREN ZUR HERSTELLUNG VON 1,4-BUTANDIOL MIT EINER APHA-FARBZAHL VON WENIGER ALS 30**
PROCESS FOR THE PREPARATION OF 1,4-BUTANEDIOL WITH AN APHA COLOUR INDEX OF LOWER THAN 30
PROCÉDÉ POUR LA PRÉPARATION DE BUTANEDIOL-1,4 AVEC UN INDICE DE COLORATION APHA INFÉRIEUR À 30

(30) Priorität: 23.10.2013 EP 13189864
(43) Veröffentlichungstag der Anmeldung: 31.08.2016
(73) Patentinhaber: BASF SE, 67056 Ludwigshafen am Rhein (DE)
(72) Erfinder: PINKOS, Rolf, 67098 Bad Dürkheim (DE); OSETSKA, Olga, 68163 Mannheim (DE); DE WISPELAERE, Irene, 68165 Mannheim (DE); DUDENHÖFFER, Stefan, 67071 Ludwigshafen (DE); WALCZUCH, Christina, 67071 Ludwigshafen (DE)
(74) Vertreter: BASF IP Association
(86) Internationale Anmeldenummer: PCT/EP2014/071886
(87) Internationale Veröffentlichungsnummer: WO 2015/058990

(56) Entgegenhaltungen:
- EP-A1- 0 000 159
- DE-A1- 19 801 089
- JP-A- H07 258 129
- JP-A- S61 197 534
- US-A- 4 032 583
- US-A- 4 358 625
- US-A- 5 209 825
- US-A1- 2003 018 224

## Beschreibung

Die Erfindung betrifft ein Reinigungsverfahren zur Herstellung von 1,4-Butandiol mit einer APHA-Farbzahl von weniger als 30 durch Destillation des 1,4-Butandiol in Gegenwart von komplexen Hydriden.

### Stand der Technik

1,4-Butandiol lässt sich auf verschiedene Wege herstellen. In WO/2013/012047 A1 und WO/2013/005748 sind einige Varianten beschrieben, beispielsweise basierend auf Diacetoxybuten, Propen, 1,4-Butindiol, Carbonsäurederivaten wie Maleinsäureanhydrid oder Bernsteinsäure sowie Fermentation direkt zu Butandiol.

US 4 032 583A beschreibt ein Verfahren zur Reinigung von 1,4-Butandiol wobei die 1,4-Butandiol haltige Mischung mit Wasser verdünnt wird, anschließend mit einem Kohlenwasserstoff extrahiert wird und danach die wässrige 1,4-Butandiol haltige Mischung destilliert wird.

JP S61 197534A beschreibt ein Reinigungsverfahren für 1,4-Butandiol, bei dem die Rohmischung mittels eines geträgerten Pt-Katalysators hydriert und anschließend destilliert wird.

DE 198 01 089A1 beschreibt ein Verfahren zur Reinigung von rohem 1,4-Butandiol, das die Schritte (i) Hydrierung des rohen 1,4-Butandiols in Gegenwart eines Hydrierkatalysators, (ii) Entfernung eines feinen Hydrierkatalysatorpulvers aus der Hydriermischung und (iii) Destillation der Mischung, von der das feine Pulver entfernt wurde in einer Destillationssäule.

US 5 209 825 A beschreibt Verfahren zur Reinigung von rohem 1,4-Butandionl mittels Destillation, bei dem hochsiedende Verunreinigungen, die auch zur Farbbildung beitragen können, in einer Bodenfraktion aus dem Gemisch abgetrennt werden und die Kopffraktion, bestehend aus 1,4-Butandiol und leichtsiedende Verunreinigungen, weiter durch Destillation getrennt werden.

In WO/2013/012047 A1 wird dabei die Hydrierung von C4-haltigen ungesättigten Ausgangsstoffen in Gegenwart von Butandiol und geringen Mengen Aminen an festen Katalysatoren mit Elementen der 9. - 11. Gruppe des Periodensystems der Elemente beschrieben. Da Amine und ungesättigte C4-Komponenten nicht die einzigen Nebenkomponenten in Butandiol-haltigen Stoffströmen sind, sondern, je nach Verfahren, auch mehr oder weniger Schwefel-haltige und weitere hydrierbare Komponenten wie z.B. Aldehyde, Halbacetale, Acetale, bzw. Ketone, Ester und Säuren sowie Anionen und Kationen enthalten, ist fraglich, ob das vorgenannte Verfahren generell gut anwendbar ist.

Schließlich ist aus wirtschaftlichen Gründen, eine möglichst lange Katalysatorlebensdauer wünschenswert. Bekanntermaßen sind v.a. Schwefelverbindungen ausgesprochene Katalysatorgifte, Säuren und Ester in Gegenwart von Wasser können die Katalysatoren so angreifen, dass deren Trägerstruktur zerstört wird, Aldehyde können CO abspalten, was die Aktivität von Katalysatoren negativ beeinflusst. Somit wird ein zwar anfänglich gut funktionierender Prozess zunehmend ineffizienter. Dies ist beispielsweise an der Zunahme an nicht hydrierten Produkten festzustellen, die dann z.B. die Farbzahl oder die Farbzahlstabilität des gewünschten Endprodukts negativ beeinflusst.

### Aufgabenstellung

Es wurde dementsprechend ein Verfahren gesucht, das auch in Gegenwart einzelner oder mehrerer der oben genannten, katalysatorschädlichen Komponenten, zuverlässig und preiswert über einen langen Zeitraum hinweg betreibbar ist.

### Beschreibung der Erfindung

Die Erfindung betrifft ein Verfahren zur Herstellung von 1,4-Butandiol mit einer APHA -Farbzahl von weniger als 30, indem 1,4-Butandiol enthaltende Stoffgemische durch Destillation des 1,4-Butandiols in Gegenwart von komplexen Hydriden behandelt werden.

Das so gewonnene 1,4-Butandiol weist hohe Reinheiten und niedrige APHA-Farbzahlen, insbesondere APHA-Farbzahlen von weniger als 30, bevorzugt von weniger als 15, insbesondere bevorzugt von weniger als 10, 9,8,7,6,5,4,3 und 2 auf, und zudem eine hohe Farbzahlstabilität, was bedeutet, dass sich die APHA-Farbzahl des Produktes auch bei Lagerung nicht nennenswert erhöht.

Als komplexe Hydride werden erfindungsgemäß BH₃, AlH₃, LiBH₄, NaBH₄, KBH₄, LiAlH₄, NaAlH₄, oder KAlH₄ eingesetzt.

Die Hydride können Zusätze enthalten wie Alkali- oder Erdalkalihydroxyde bzw. z.B. Li-, La- und Ce-Salze in Form der Halogenide, Sulfate, Phosphate oder Carboxylate.

Besonders bevorzugt sind Natriumborhydrid, beispielsweise in Form von Borol™ und Lithiumaluminiumhydrid. Ganz besonders bevorzugt ist NaBH₄, beispielsweise in Form alkalischer wässriger Lösungen, wie sie von Rohm und Haas unter der Bezeichnung "Borol™-Lösung" oder von Dow Chemical Company unter der Bezeichnung "VenPure™- Lösung" vertrieben werden. Dabei handelt es sich um eine wässrige Lösung von 25-40 Gew-% NaOH und 10-12,5 Gew-% NaBH₄.

Die komplexen Hydride werden beispielsweise in Mengen zwischen 1 und 50000 ppm, bevorzugt zwischen 50 bis 25000 ppm, besonders bevorzugt zwischen 100 und 10000 ppm bezogen auf die Menge an zu reinigendem Butandiol eingesetzt.

Nach Zusatz der komplexen Hydride, weist die Lösung, sofern ein Wassergehalt > 10 % vorliegt, einen pH von 4 bis 14 auf. Bevorzugt liegt der pH zwischen 5 und 12, ganz besonders bevorzugt zwischen 6 und 10. Sofern der Wassergehalt im zu reinigenden Butandiol-haltigem Strom unter 10 % liegt, kann, zur Messung des pH-Wertes, das Gemisch mit Wasser verdünnt werden.

Die komplexen Hydride können in Substanz oder in Lösung oder als Suspension eingebracht werden. Als Lösemittel sollte eines verwendet werden, das mit dem Hydrid keines bzw. nur wenig Wasserstoff entwickelt. Bevorzugt sind Wasser, das zu reinigende Butandiol, Ether wie z.B. Tetrahydrofuran und Diethylether. Besonders bevorzugt ist Wasser, das zusätzlich Alkalihydroxide wie NaOH enthalten kann, bzw. als bereits zubereitete Borol™-Lösung.

Die Zugabe des komplexen Hydrids, das zum zu reinigenden Butandiol hinzugefügt wird, kann bei Temperaturen zwischen 10 und 250 °C, bevorzugt zwischen 20 und 170°C, besonders bevorzugt zwischen 35 und 100°C erfolgen, je nach Ort der Zugabe.

Sofern das komplexe Hydrid zum zu reinigenden Butandiol enthaltenden Stoffgemisch vor der zumindest einen Destillationskolonne zugegeben wird, beträgt die mittlere Verweilzeit in dem Behältnis vor der Kolonne zumindest 0,1 h. Bevorzugt sind 0,5 h, besonders bevorzugt sind 1 h.

Im Folgenden wird auf die Destillation im Allgemeinen eingegangen, um dann im Anschluss zu verdeutlichen, wo und wie man die Hydride einsetzen kann.

Das Ausgangsmaterial mit den Butandiol enthaltenden Stoffgemischen, das auch als Roh-Butandiol bezeichnet wird, enthält im Allgemeinen neben 1,4-Butandiol noch Leicht- und Schwersieder, die möglichst weitgehend abgetrennt werden müssen. Leichtsieder sind, je nach Verfahren, beispielsweise Wasser, Methanol, Ethanol, Propanol, Butanol, Essigsäure, Acetate, Formaldehydacetale, gamma-Butyrolacton, Acetaldehyd, Acetaldehydacetale, z.B. das mit 1,4-Butandiol, andere Ester, Ketone, N-haltige Verbindungen, 4-Hydroxybutyraldehyd, isomere Butandiole, Ethylenglykol, 1,2- bzw. 1,3-Propylenglkol, THF.

Schwersieder sind je nach Herstellverfahren beispielsweise Pentandiol und Hexandiole, dimeres Butandiol, Ester aus Butandiol und gamma-Butyrolacton, Hydroxycarbonsäuren wie z.B. Milchsäure oder Äpfelsäure, Bernsteinsäure oder deren Ester, Glycerin, Aminosäuren, Amide, Kohlenhydrate und Derivate bzw. Abbauprodukte von Kohlenhydraten .

Daraus ergibt sich die Aufarbeitung mittels Destillation in mindestens einer Kolonne. Bevorzugt ist dabei eine kontinuierliche Aufarbeitung. Wird tatsächlich nur eine Kolonne eingesetzt, so werden Leichtsieder, zumindest zum Teil, über Kopf, Hochsieder über Sumpf und Butandiol als Seitenabzug (Gas- oder Flüssigabzug) erhalten.

In einer besonders geeigneten Ausführungsform kommt hierbei eine sogenannte Trennwandkolonne zum Einsatz. Je nach dem Gehalt und der Natur der von Butandiol zu trennenden Komponenten, ist es meist vorteilhaft, mehrere Kolonnen zu verwenden. Ist als ein wesentlicher Bestandteil an den Leichtsiedern Wasser vorhanden, so wird dieses zusammen mit ggf. anderen Leichtsiedern in mindestens einer Kolonne bei Drücken von 0,05 bis 5 bar und Sumpftemperaturen zwischen 80 und 200°C abgetrennt.

Ist viel Wasser vorhanden, beispielsweise mehr als 40 % Gew.%-Anteil an der Butandiol-haltigen Mischung, so kann es vorteilhaft sein, das Wasser in mindestens zwei Kolonnen abzutrennen, wobei dabei die erste bei höheren Drücken z.B. zwischen 1 und 5 bar absolut betrieben wird, die zweite dann bei geringeren Drücken, z.B. bei 100 - 1000 mbar absolut.

In einer gegebenenfalls zweiten Kolonne wird reines Butandiol dann von Schwersiedern getrennt, wobei Butandiol dabei über Kopf oder als Seitenabzug, ggf. in einer Trennwandkolonne, erhalten wird.

In einer weiteren bevorzugten Ausführungsform, werden die komplexen Hydride zum aufzuarbeitenden Butandiol hinzugefügt, von Leichtsiedern wie Wasser destillativ befreit und das Hydrid enthaltende Butandiol über eine einfache Verdampfung in Hochsieder, die das Hydrid enthalten und Butandiol aufgetrennt. Das Butandiol kann dann wie oben beschrieben, weiter destillativ gereinigt werden. Die Verdampfung erfolgt beispielsweise in einem Dünnschichtverdampfer (Sambay®-Verdampfer, einem Fallfilmverdampfer oder anderen, dem Fachmann bekannten, Verdampfern.

Sollte nur wenig Butandiol aufgereinigt werden, so ist dies auch durch absatzweise Destillation möglich. Es ist je nach Erfordernissen auch eine Kombination von absatzweiser und kontinuierlicher Destillation möglich. Bei der absatzweisen Destillation werden im Allgemeinen zuerst die Leichtsieder abdestilliert, anschließend das Butandiol. Die Hochsieder verbleiben im Sumpf und werden anschließend entsorgt. Die Leichtsieder werden im Allgemeinen bei höherem Druck abdestilliert als Butandiol. Beispielsweise wird Wasser und andere Leichtsieder bei Umgebungsdruck abdestilliert. Sobald die Destillationsleistung sinkt, wird kontin. der Druck gesenkt und/oder die Temperatur im Sumpf erhöht. So destilliert dann beispielsweise reines Butandiol bei Drücken um 45 mbar absolut und Sumpftemperaturen von ca. 152°C ab.

Vorteilhaft werden die Destillationen in Kolonnen mit die Trennung verstärkenden Einbauten vorgenommen, v.a. bei der Reindestillation des Butandiols. Diese Einbauten sollten einer theoretischen Bodenzahl von zumindest 5 entsprechen, bevorzugt sind 10, besonders bevorzugt 15. Die Einbauten können beispielsweise eine lose Schüttung von Füllkörpern aus Metall, Keramik oder Teflon sein, Ventilböden, Glockenböden oder Packungen, wie sie im Handel erhältlich sind.

Für ein optimales Gelingen des erfindungsgemäßen Verfahrens ist es wichtig, dass die Destillationen, v.a. die Reindestillation von Butandiol, weitgehend unter Luftausschluss stattfindet. Dabei beträgt das molare Verhältnis von Sauerstoff zu 1,4-Butandiol weniger als 1 : 100, bevorzugt weniger als 1 : 1000, besonders bevorzugt weniger als 1 : 10000. Zur Vermeidung von Sauerstoff empfiehlt es sich möglichst dichte Kolonnen zu verwenden. Ist zu viel Sauerstoff vorhanden, kann einerseits das Reinheitsziel des Butandiols, z.B. durch Oxidationsreaktionen, verfehlt werden, andererseits ist mit erhöhten Farbzahlen zu rechnen. Ferner kann Sauerstoff die Wirkung der Hydride beeinträchtigen bzw. durch Oxidation der Hydride Ablagerungen in den Kolonnen und Verdampfern generieren, die aufwändige Reinigungen der Kolonnen bedingen würden.

Je geringer der Destillationsdruck, desto mehr muss auf die Dichtigkeit der verwendeten Destillationseinrichtung geachtet werden. Erhöhte Dichtigkeit bei Kolonnen erreicht man z.B. durch Schweißlippendichtungen, Dichtungen mit Kammprofilen, durch Verwendung von besonders glatten Dichtflächen, durch Vermeidung einer Vielzahl von Flanschen oder Zugangsstellen in den Kolonnen wie z.B. zur Messung von Druck, Temperatur oder Schaugläser usw.

Eine weitere Möglichkeit zur Vermeidung von Sauerstoff ist, die Destillationseinheit mit einem äußeren Mantel zu versehen, der beispielsweise mittels Stickstoff oder Argon inertisiert wird. Ferner können Flansche auch zugeschweißt werden.

Eine Möglichkeit zur Messung von Sauerstoff besteht darin, dass das Abgas der Vakuumeinheit aufgefangen wird und das enthaltene Gasgemisch auf seine Zusammensetzung hin analysiert. Dabei wird eine Aussage über den Sauerstoffeintrag in die Kolonne am besten dadurch erhalten, dass die Kolonne unter den bevorzugten Bedingungen betrieben wird, allerdings ohne Zulauf.

Die komplexen Hydride können bereits vor der Leichtsiederabtrennung oder aber danach eingesetzt werden. Es ist beispielsweise möglich, NaBH4 in Form von Formkörpern einzusetzen, über das der Butandiol-haltige Strom teilweise oder in Gänze bei Temperaturen von 10 - 80 °C geleitet wird. Dabei nimmt der Produktstrom das Hydrid in gelöster Form teilweise auf und gelangt so in die Destillation. Das Hydrid kann aber auch als Pulver dem Produktstrom zugemischt werden, so dass es in der Destillation als homogenes Gemisch vorliegt. Ferner kann das Hydrid separat in einem Lösemittel wie Wasser oder Methanol oder THF gelöst werden und als homogene Lösung dem Butandiol-haltigen Strom zugeführt werden. Dabei ist eine bevorzugte Ausführungsform die Zuführung von Borol™ ein kommerziell verfügbares Produkt als Mischung von NaBH4, Wasser und NaOH.

Bevorzugte Stellen, an denen das komplexe Hydrid ins Verfahren eingebracht werden kann sind der Leichtsieder-haltige Zulauf, der Sumpf der Leichtsiederabtrennkolonne, der Zulauf der Butandiol-Reinkolonne und der Sumpf der Butandiol-Reinkolonne. Besonders bevorzugt sind der Leichtsieder-haltige Zulauf bzw. der Zulauf zur Butandiol-Reinkolonne.

Eine Besonderheit des Verfahrens ist der Umgang mit dem Hydrid-haltigen Sumpfprodukt der Butandiol-Reinkolonne. Bei Sumpftemperaturen von 130 - 230°C, bevorzugt 150 - 190°C wird der Restbutandiol-Gehalt im Sumpf auf 1 - 90 Gew.%, bevorzugt auf 5 - 70 Gew.%, besonders bevorzugt auf 10 - 50 Gew.% eingestellt. Nach Abkühlen des Sumpfes auf unter 100°C wird dieser mit Wasser versetzt. Vorzugsweise wird dazu das Leichtsiederprodukt oder ein Teilstrom dessen, verwendet. Wird die Verdünnung nicht durchgeführt, besteht die Gefahr des Ausfallens eines Feststoffes. Der Grad der Verdünnung zumindest 1 Teil Wasser auf 2 Teile Sumpf, bevorzugt zumindest 1 : 1, besonders bevorzugt zumindest 2 : 1.

Sofern man Resthydride beseitigen möchte, kann der Sumpf angesäuert werden. Dazu sind im Prinzip alle Säuren möglich, bevorzugt die, die sich mit dem ggf. mit Wasser versetztem Sumpf mischen, wie z.B. (ggf. mit Wasser verdünnte) Ameisensäure, Essigsäure, Schwefelsäure oder Salzsäure.

Das nach dem erfindungsgemäßen Verfahren hergestellte Butandiol weist besonders hohe Reinheiten auf und ist auch über längere Zeiten Farbzahl-stabil. Die Reinheit wird wie üblich über Gaschromatographie bestimmt und liegt über 99 %, bevorzugt über 99,6 %, besonders bevorzugt über 99,8 %.

Ein weiteres Kennzeichen des erfindungsgemäßen Verfahrens ist der niedrige Gehalt an 4-Hydroxy-1-butoxy-THF im destillierten Butandiol. Dieser liegt im Allgemeinen unter 3000 ppn, bevorzugt unter 1500 ppm, besonders bevorzugt unter 800 ppm.

Die Farbzahlen (APHA-Hazen-Farbzahl) liegen unter 30 APHA, bevorzugt unter 20 APHA, besonders bevorzugt unter 10 APHA. Die APHA-Farbzahl ist ein von der American Public Health Association (APHA) herausgegebenes Prüfverfahren zur Farbmessung in Flüssigkeiten. Als Kalibrierungsstandard wird nach ISO 6271 eine Kobalt-Platinat-Lösung verwendet. Die Messung der APHA-Farbzahl kann leicht in speziellen Photometern, z.B. solchen der Firma Chemtronic Waltemode GmbH, erfolgen.

Acetal bedeutet im Folgenden 4-Hydroxy-1-butoxy-THF.

Alle Destillationen wurden in einer Batch-Kolonne durchgeführt. Die Kolonne hatte einen Durchmesser von ca. 2,7 cm und war auf einer Länge von 80 cm mit 3 - 5 mm Raschigringen gefüllt. Am Kopf der Kolonne befand sich ein Rücklaufteiler, der so eingestellt wurde, dass 1 Destillatteil abgezogen, 2 Destillatteile in die Kolonne rückgeführt wurden.

Das erfindungsgemäße Verfahren erlaubt die Herstellung von hochgereinigtem 1,4-Butandiol, das eine APHA-Farbzahl von weniger als 30 aufweist. Weiterhin ist dieses so hergestellte 1,4-Butandiol auch lagerstabil, d.h. auch bei einer Lagerung beispielsweise über 24 Stunden bei erhöhter Temperatur (50°C) nimmt die APHA-Farbzahl nicht nennenswert zu.

### Experimenteller Teil

### Beispiel 1:

In einen 1 Liter Rundkolben wurden 750 g eines Rohbutandiols vorgelegt, das neben ca. 49 % 1,4-Butandiol, ca. 45 % Wasser, ca. 0,3 % gamma-Butyrolacton, 0,15 % Acetal, 0,05 % 4-Hydroxybutyraldehyd, ca. 1 % Methanol, ca. 1,1 % Propanol, ca. 1,3 % n-Butanol, ca. 0,1 % Formaldehydacetale und Halbacetale, ca. 0,1 % 2-Methyl-1,4-butandiol sowie einige z.T. unbekannte Hoch- und Leichtsieder enthielt. Das Gemisch wies eine Farbzahl von 175 APHA und einen pH von 7,1 auf. Unter Inertgas (Stickstoff) wurden 5 g einer Borol™-Lösung (von Firma Dow-Chemicals, VenPlus Solution) zugefügt. Aus der Mischung wurden ca. 94 g Leichtsieder bei anfänglich 500 mbar, dann bei 100 mbar und Sumpftemperaturen von 112 - 134°C abdestilliert. Nach Wechsel der Fraktion, wurde die Sumpftemperatur auf ca. 150 °C erhöht, und bei 50 mbar ca. 65 g Zwischenfraktion (ca. 32 % Wasser, GC-Gehalt, wasserfrei gerechnet, an Butandiol: 98,1 %, Farbzahl 6 APHA) abdestilliert. Danach wurden bei ca. 40 mbar ca. 520 g Hauptlauf bei ca. 150 °C abdestilliert. Das 1,4-Butandiol des Hauptlaufs hatte eine GC-Reinheit von 99,85 %, 2-Methylbutandiol 0,092 %, Acetalgehalt lag bei 0,077 %, gamma-Butyrolacton war nicht nachweisbar. Die Farbzahl lag bei 8 APHA. Die Probe wurde in eine Blechdose (Material 1.0425) abgefüllt und bei 50 °C gelagert. Nach 24 h lag die Farbzahl unverändert bei 8 APHA.

### Vergleichsbeispiel 1:

Beispiel 1 wurde wiederholt, allerdings ohne die Zugabe von Borol™. Nach Abtrennen der Hauptmenge an Leichtsieder und Wasser, wurden ca. 85 g Zwischenlauf erhalten (Farbzahl 7 APHA, Wassergehalt ca. 80 %, GC-Gehalt, wasserfrei gerechnet, an Butandiol: ca. 60 %). Als Hauptlauf wurden ca. 4 g erhalten. Das 1,4-Butandiol des Hauptlaufs hatte eine GC-Reinheit von 99,54 %, 2-Methylbutandiol 0,095 %, Acetalgehalt lag bei 0,219 %, gamma-Butyrolacton 0,014 %. Die Farbzahl lag bei 6 APHA. Die Probe wurde in eine Blechdose (Material 1.0425) abgefüllt und bei 50 °C gelagert. Nach 24 h lag die Farbzahl bei 35 APHA.

### Beispiel 2:

In einen 1 Liter Rundkolben wurden 750 g eines Rohbutandiols vorgelegt, das neben ca. 84 % 1,4-Butandiol, ca. 15 % Wasser, ca. 0,01 % gamma-Butyrolacton, ca. 0,15 % Acetal, ca. 0,01 % Acetaldehyd sowie dessen Acetal bzw. Halbacetal mit Butandiol sowie einige weitere unbekannte Hoch- und Leichtsieder enthielt. Das Gemisch wies eine Farbzahl von 75 APHA und einen pH von 9 auf. Unter Inertgas (Stickstoff) wurden 5 g einer Borol™-Lösung (von Firma Dow-Chemicals, VenPlus Solution) zugefügt. Aus der Mischung wurden ca. 94 g Leichtsieder bei anfänglich 500 mbar, dann bei 100 mbar und Sumpftemperaturen von 112 - 134°C abdestilliert. Nach Wechsel der Fraktion, wurde die Sumpftemperatur auf ca. 150 °C erhöht, und bei 50 mbar ca. 65 g Zwischenfraktion (ca. 32 % Wasser, GC-Gehalt, wasserfrei gerechnet, an Butandiol: 98,1 %, Farbzahl 6 APHA) abdestilliert. Danach wurden bei ca. 40 mbar ca. 520 g Hauptlauf bei ca. 150 °C abdestilliert. Das 1,4-Butandiol des Hauptlaufs hatte eine GC-Reinheit von 99,895 %, Acetalgehalt lag bei 0,071 %, gamma-Butyrolacton war nicht nachweisbar. Die Farbzahl lag bei 6 APHA. Die Probe wurde in eine Blechdose (Material 1.0425) abgefüllt und bei 50 °C temperiert. Nach 24 h lag die Farbzahl unverändert bei 6 APHA.

### Vergleichsbeispiel 2:

Beispiel 2 wurde wiederholt, allerdings ohne die Zugabe von Borol™ (pH-Wert ca. 6,5). Nach Abtrennen der Hauptmenge an Wasser, wurden ca. 78 g Zwischenlauf erhalten (Farbzahl 15 APHA, Wassergehalt ca. 39 %, GC-Gehalt, wasserfrei gerechnet, an Butandiol: 97,5 %). Als Hauptlauf wurden ca. 529 g erhalten. Das 1,4-Butandiol des Hauptlaufs hatte eine GC-Reinheit von 99,693 %, Acetalgehalt lag bei 0,175 %, gamma-Butyrolacton 0,014 %. Die Farbzahl lag bei 6 APHA. Die Probe wurde in eine Blechdose (Material 1.0425) abgefüllt und bei 50 °C temperiert. Nach 24 h lag die Farbzahl bei 51 APHA.

### Beispiel 3:

In einen 1 Liter Rundkolben wurden 750 g eines Rohbutandiols vorgelegt, das neben ca. 98 % 1,4-Butandiol, ca. 0,5 % Wasser, ca. 0,5 % gamma-Butyrolacton, 0,13 % Acetal sowie einige weitere unbekannte Hoch- und Leichtsieder enthielt. Das Gemisch wies eine Farbzahl von 25 APHA auf. Unter Inertgas (Stickstoff) wurden 0,5 g LiAIH4 zugefügt. Aus der Mischung wurden bei einer Sumpftemperatur von ca. 150 °C und 50 mbar ca. 50 g abdestilliert. Diese Fraktion enthielt ca. 8 % Wasser, GC-Gehalt, wasserfrei gerechnet, an Butandiol 93,2 %, Farbzahl 8 APHA). Danach wurden bei ca. 40 mbar ca. 620 g Hauptlauf bei ca. 150 °C abdestilliert. Das 1,4-Butandiol des Hauptlaufs hatte eine GC-Reinheit von 99,912 %, Acetalgehalt lag bei 0,054 %, gamma-Butyrolacton war nicht nachweisbar. Die Farbzahl lag bei 5 APHA. Die Probe wurde in eine Blechdose (Material 1.0425) abgefüllt und auf 50 °C aufgeheizt. Nach 24 h lag die Farbzahl unverändert bei 5 APHA.

### Vergleichsbeispiel 3:

Beispiel 3 wurde wiederholt, allerdings ohne die Zugabe von LiAlH4. Als erste Fraktion wurden ca. 45 g erhalten (Farbzahl 9 APHA, Wassergehalt ca. 9 %, GC-Gehalt, wasserfrei gerechnet, an Butandiol: 92,5 %). Als Hauptlauf wurden ca. 623 g erhalten. Das 1,4-Butandiol des Hauptlaufs hatte eine GC-Reinheit von 99,731 %, Acetalgehalt lag bei 0,151 %, gamma-Butyrolacton 0,024 %. Die Farbzahl lag bei 6 APHA. Die Probe wurde in eine Blechdose abgefüllt. Nach 24 h lag die Farbzahl bei 12 APHA.

## Patentansprüche

1. Verfahren zur Herstellung von 1,4-Butandiol mit einer APHA-Farbzahl von weniger als 30, in dem 1,4-Butandiol enthaltende Stoffgemische durch Destillation des 1,4-Butandiol in Gegenwart von komplexen Hydriden aus der Gruppe, die von BH₃, AlH₃, LiBH₄, NaBH₄, KBH₄, LiAlH4, NaAlH₄, KAlH₄ gebildet wird, behandelt werden.

2. Verfahren nach Anspruch 1, **dadurch gekennzeichnet, dass** als komplexes Hydrid NaBH₄ eingesetzt wird.

3. Verfahren nach Anspruch 2, **dadurch gekennzeichnet, dass** NaBH₄ als wäßrige alkalische Lösung eingesetzt wird.

4. Verfahren nach Anspruch 1, **dadurch gekennzeichnet, dass** die Destillation des 1,4-Butandiol kontinuierlich betrieben wird.

5. Verfahren nach Anspruch 4, **dadurch gekennzeichnet, dass** die Destillation mit einer Trennwandkolonne betrieben wird.

6. Verfahren nach den Ansprüchen 1 bis 5, **dadurch gekennzeichnet, dass** der pH-Wert der Mischung aus1,4- Butandiol enthaltenem Stoffgemisch und Hydrid, bei einem Mindestwassergehalt von 10 % einen pH von 4 bis 14 aufweist.

## Claims

1. A method for preparing 1, 4-butanediol having an APHA color index of less than 30 by treating substance mixtures comprising 1,4-butanediol by distillation of the 1,4-butanediol in the presence of complex hydrides from the group formed by BH₃, ALH₃, LiBH₄, NaBH₄, KBH₄, LiAlH₄, NaAlH₄, KAlH₄.

2. The method according to claim 1, wherein the complex hydride used is NaBH₄.

3. The method according to claim 2, wherein NaBH₄ is used as an aqueous alkaline solution.

4. The method according to claim 1, wherein the distillation of the 1,4-butanediol is operated continuously.

5. The method according to claim 4, wherein the distillation is operated using a dividing wall column.

6. The method according to claims 1 to 5, wherein the pH of the mixture of substance mixture comprising 1,4-butanediol and hydride has a pH of 4 to 14 at a minimum water content of 10%.

## Revendications

1. Procédé pour la préparation de 1,4-butanediol présentant un indice de couleur APHA inférieur à 30, dans lequel des mélanges de produits contenant du 1,4-butanediol sont traités par distillation du 1,4-butanediol en présence d'hydrures complexes du groupe qui est formé par BH₃, AlH₃, LiBH₄, NaBH₄, KBH₄, LiAlH₄, NaAlH₄, KAlH₄.

2. Procédé selon la revendication 1, **caractérisé en ce que** NaBH₄ est utilisé en tant qu'hydrure complexe.

3. Procédé selon la revendication 2, **caractérisé en ce que** NaBH₄ est utilisé en tant que solution aqueuse alcaline.

4. Procédé selon la revendication 1, **caractérisé en ce que** la distillation du 1,4-butanediol fonctionne en continu.

5. Procédé selon la revendication 4, **caractérisé en ce que** la distillation fonctionne avec une colonne à paroi de séparation.

6. Procédé selon les revendications 1 à 5, **caractérisé en ce que** la valeur de pH du mélange d'hydrure et de mélange de produits contenant du 1,4-butanediol, présente, à une teneur minimum en eau de 10 %, un pH de 4 à 14.
